# EUROPÄISCHE PATENTANMELDUNG

(11) **EP 1 300 413 A1**
(43) Veröffentlichungstag der Anmeldung: **09.04.2003**
(21) Anmeldenummer: 01123860.7
(22) Anmeldetag: 05.10.2001
(51) Int. Cl.: C07H 15/04

(54) **Verfahren zum Herstellen von Glykosiden**

(71) Anmelder: Haltermann GmbH, 20095 Hamburg (DE)
(72) Erfinder: Hildebrandt, Rainer, 21149 Hamburg (DE); Vollmer, Hans-Jürgen, Dr., 22149 Hamburg (DE); Holst, Anke, 21647 Moisburg (DE); Höltmann, Wilhelm, Dr., 21220 Seevetal (DE)
(74) Vertreter: Glawe, Delfs, Moll & Partner

(57) **Zusammenfassung**

Gegenstand der Erfindung ist ein Verfahren zum Herstellen von Ethern aus Zuckern und C₁-C₈-Alkoholen. Erfindungsgemäß wird die Veretherung durch Reaktivdestillation an einem heterogenen Katalysator vorgenommen. Die Erfindung erlaubt die Herstellung von Glykosiden hoher Reinheit mit guter Ausbeute.

## Beschreibung

Die Erfindung betrifft ein Verfahren zum Herstellen von Glykosiden (Zuckerethern) aus Sacchariden (Zuckern) und C₁-C₈-Alkoholen.

Mono-, Di-, Tri-, Oligo- oder Polysaccharide (nachfolgend allgemein als Zucker oder Saccharide bezeichnet) werden insbesondere in der Nahrungsmittelindustrie für eine Reihe von Zwecken verwendet. Eine Weiterverarbeitung geschieht häufig über die Stufe von Glykosiden oder Zuckerethern, in der Hydroxygruppen der Saccharide mit kurzkettigen Alkoholen als Schutzgruppe verethert werden. Diese Veretherung erfolgt üblicherweise in flüssiger Phase in Anwesenheit saurer Katalysatoren. Die Reaktion verläuft langsam und mit geringer Ausbeute, zudem kommt es durch in der Reaktionsmischung verbleibende Katalysatorbestandteile zu einer Vielzahl unerwünschter Nebenreaktionen.

Der Erfindung liegt die Aufgabe zugrunde, ein eingangs genanntes Verfahren zu schaffen, das eine Herstellung der genannten Glykoside in guter Ausbeute und mit hoher Reinheit gestattet.

Die Erfindung löst diese Aufgabe dadurch, daß die Veretherung durch Reaktivdestillation an einem heterogenen Katalysator erfolgt.

Der Begriff Reaktivdestillation bezeichnet die Kombination von chemischer Reaktion (hier Veretherung) und destillativer Stofftrennung. Entscheidend ist, daß unmittelbar im Anschluß an die katalysierte Reaktion eine destillative Stofftrennung der Produkte und ggf. verbleibender Edukte der Reaktion erfolgt. Anders als bei einer homogenen Katalyse mit einem flüssigen Katalysator wird somit das Reaktionsprodukt unmittelbar nach der Reaktion von dem heterogenen Katalysator getrennt.

Als Katalysator eignet sich jedes Material, das die Veretherungsreaktion zu katalysieren vermag. Bevorzugt werden saure Katalysatoren verwendet. Der Katalysator ist heterogen. Dies bedeutet, daß er in einem anderen Aggregatzustand vorliegt als Edukte und Produkte der Veretherungsreaktion. In der Regel wird ein Festphasenkatalysator verwendet.

Bevorzugt werden Monosaccharide wie bspw. Glucose als Edukt eingesetzt. 60 - 80 %iger Glucosesirup mit einem Wasseranteil von 20 - 40 % ist bspw. bei 80°C hinreichend flüssig und pumpbar, so daß dieser Sirup in die Kolonne eingespeist werden kann. Im Rahmen der Erfindung werden die Begriffe Glykoside und Zuckerether synonym verwendet und umfassen alle Ether von Mono-, Di-, Tri-, Oligo- oder Polysacchariden (Zuckern) mit den im Anspruch genannten C₁ - C₈-Alkoholen. Bevorzugt wird ein Ether mit einem C₂ - C₈ Alkohol hergestellt, besonders bevorzugt mit einem C₂ - C₄ Alkohol. Der Alkohol ist bevorzugt ein primärer oder sekundärer Alkohol, der ausgewählt sein kann aus der Gruppe bestehend aus Ethanol, n-Propanol, Isopropanol, n-Butanol, 2-Butanol und sec-Butanol.

Der Katalysator ist bevorzugt in der Destillations- bzw. Rektifikationskolonne fixiert. Bevorzugt wird im Rahmen der Erfindung eine gepackte Kolonne (Definition siehe Ullmanns Encyclopedia of Industrial Chemistry, 5. Auflage, Band B3, Seite 4-71) verwendet. Die gesamte Kolonnenpackung oder ein Teil davon kann mit einem heterogenen Katalysator versehen oder durch diesen ersetzt werden.

Als Träger zur Fixierung des Katalysators kann eine in der Kolonne befestigte strukturierte Packung verwendet werden wie beispielsweise die Packung Katapak®-S der Sulzer Chemtech AG. Diese Packung besteht aus lagenweise angeordnetem Maschendrahtgewebe, zwischen die Gewebelagen kann der Katalysator in Taschen eingelagert und dadurch fixiert werden.

Als Katalysator findet bevorzugt ein saures Ionenaustauscherharz wie beispielsweise das makroporöse saure Ionenaustauscherharz Amberlyst®15, Firma Rohm und Haas, Verwendung. Es können handelsübliche Pellets dieses Katalysators mit einem Durchmesser zwischen 0,35 und 1,2 mm verwendet werden.

Die gewünschte Kontaktzeit der Edukte in der Kolonne kann durch Variation der Destillations-/Rektifikationsparameter gesteuert werden. Das höhersiedende oder nicht destillierbare Edukt (in der Regel der Zucker) wird vorzugsweise oberhalb des Katalysators in die Destillationskolonne eingespeist (vorzugsweise als wäßrige Lösung oder Sirup), das niedrigersiedende Edukt unterhalb dieses Katalysators. Die Edukte kommen so im Gegenstrom im Bereich der Katalysatorpackung miteinander zur Reaktion. Die Reaktionsparameter können ferner beeinflußt werden durch die Wahl des Rücklaufverhältnisses der Kolonne, der Kolonnentemperatur und (davon abhängig) des Drucks in der Kolonne.

Ein besonderer Vorteil der Reaktivdestillation ist, daß bei der Veretherung entstehendes Reaktionswasser destillativ sofort abgetrennt wird und damit das Reaktionsgleichgewicht auf die Seite des Ethers verschoben wird. Ferner wird auf diese Weise ein ggf. in den Edukten enthaltener Wasseranteil entfernt. In der Regel müssen die als Edukte verwendeten Saccharide einen Wasseranteil von bspw. etwa 20 % aufweisen, damit sie als Flüssigphase in die Reaktionskolonne eingespeist werden können.

Bei Bedarf kann zur Wasserabtrennung zusätzlich ein geeignetes Schleppmittel verwendet werden, mit dem in den Edukten vorhandenes Wasser sowie Reaktionswasser als Azeotrop abdestilliert. Unter Umständen kann als Schleppmittel sogar der zur Veretherung verwendete Alkohol verwendet werden. Bei der Herstellung eines Isopropylethers kann im Überschuß eingesetzter Isopropanol gleichzeitig als Schleppmittel für Wasser dienen.

Die Reaktionstemperatur am Katalysator kann bei 60 - 200°C, vorzugsweise bei 60 - 150°C, weiter vorzugsweise 70 - 100°C liegen. Diese Temperatur hängt ab von den Siedepunkten der in der Kolonne destillierenden flüssigen bzw. gasförmigen Produkte, diese können ggf. variiert werden durch Einstellen eines Über- oder Unterdrucks in der Kolonne. Eine Obergrenze der Temperatur wird ferner bestimmt durch das verwendete Katalysatormaterial. Beispielsweise sind styrolbasierte Katalysatoren nur bis etwa 95 - 100°C temperaturstabil, bei höheren Temperaturen spalten sie Schwefelsäure ab. Silicobasierte Katalysatoren sind häufig temperaturstabiler bis beispielsweise etwa 200°C. Den Druck der Kolonne wählt man in der Regel so, daß der eingesetzte Alkohol bei einer Temperatur siedet, die unterhalb einer etwaigen Zersetzungstemperatur des verwendeten heterogenen Katalysators liegt.

Der bei der Reaktion erhaltene schwerflüchtige Ether wird als Sumpfprodukt aus der Kolonne abgezogen. Bei temperaturempfindlichen Ethern kann die Sumpftemperatur unter Umständen so hoch sein, daß Zersetzungsreaktionen auftreten. In diesem Fall kann man zur Senkung der Sumpftemperatur ein niedriger siedendes Lösungsmittel in dem Sumpf schneiden, das in einem nachfolgenden Destillationsschritt entfernt werden kann.

Die erfindungsgemäße Verwendung eines heterogenen Katalysators im Rahmen einer Reaktivdestillation hat den zusätzlichen Vorteil, daß in den Edukten ggf. enthaltene Verunreinigungen von Metallionen unter Deaktivierung des Katalysators aus der Flüssigphase entfernt werden.

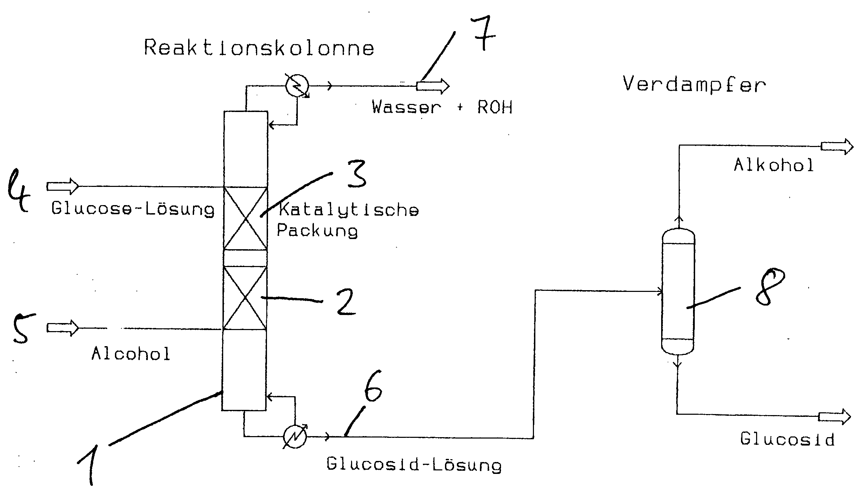

Die Erfindung wird nachfolgend anhand von Ausführungsbeispielen beschrieben. Die Figur zeigt schematisch eine Anlage zur Durchführung des erfindungsgemäßen Verfahrens.

Eine gepackte Rektifikationskolonne 1 weist mehrere Sektionen auf. Zwei mit den Bezugsziffern 2 und 3 bezeichnete Sektionen weisen eine katalytische Packung auf, es kann sich dabei um die oben erwähnte Katapak®-S der Sulzer Chemtech AG handeln. Diese katalytische Packung ist mit einem sauren Ionenaustauscher in Form von Pellets oder Kügelchen gefüllt.

In die Kolonne 1 wird die Glucose oder ein anderer Zucker bei 4 in flüssiger Form oberhalb der katalytischen Packung und der Alkohol bei 5 unterhalb der katalytischen Packung eingespeist. Am Kolonnenkopf wird bei 7 Wasser aus Einsatzwaren und Reaktionswasser oder ein azeotropes Wasser-Alkohol-Gemisch abgezogen. Zur Entwässerung kann ggf. auch ein Schleppmittel verwendet werden.

Der Rohether wird als Lösung im verwendeten Alkohol als Sumpfprodukt bei 6 abgezogen. Der Rohether enthält in der Regel 70-95% Monoglucosid und weniger als 1-2% unumgesetzte Glucose. Durch Erhöhung des Alkoholgehaltes kann die Sumpftemperatur abgesenkt werden, um eine thermische Schädigung des erhaltenen Glycosids zu vermeiden und um Folgereaktionen zu unterdrücken. Dieses in Alkohol gelöste Glycosid kann in einer weiteren Destillationsanlage 8 (Verdampfer, Eindicker, Fallfilmverdampfer oder Dünnschichtverdampfer) unter Vakuum schonend aufkonzentriert werden. Abdestillierter Alkohol kann wieder in der Reaktionskolonne eingesetzt werden.

Im Stand der Technik muß der verwendete Katalysator vor einer Aufkonzentrierung neutralisiert oder durch andere Maßnahmen entfernt werden. In der Regel werden deshalb zahlreiche Nebenreaktionen zu mehrfach substituierten Glycosiden beobachtet sowie Di- und Triglucoside als Nebenprodukte erhalten.

Sämtliche Prozentangaben in den Beispielen sind Gewichtsprozente, soweit nicht anders angegeben.

### Beispiel 1

### Veretherung von Glucose mit 2-Butanol

| | |
|---|---|
| Anlage | Kontinuierliche Destillationsanlage mit fünf Sektionen. Unterste Sektion und Sektion 4 und 5 mit 40 mm Durchmesser mit Sulzer CY® Packung, Sektionen 2 und 3 mit 70 mm Durchmesser und katalytischer Packung. Phasentrenner am Kopf mit Rückführung der organischen Phase über Sektion 4. Glucoseeinsatz über Sektion 3 und 2-Butanoleinsatz über Sektion 1. |
| Versuchsdauer | 8 Stunden |
| Druck | 800 mbar |
| Differenzdruck | 2 mbar |
| Rückfluß | 1 : 3 bis 1 : 6 |
| Einsätze | |
| 2- Butanol | Über 1. Schuß mit 90°C 340 - 590 g/h |
| Glucose 63 %ig | Über 3. Schuß mit 60°C 80 - 170 g/h |
| Abläufe | |
| Wasserphase | 54 - 83 g/h |
| Sumpf | 414 - 653 g/h |
| Temperaturen | |
| Kopf | 77 - 78°C |
| über 4. Schuß | 78 - 88°C |
| über 3. Schuß | 89 - 91°C |
| über 2. Schuß | 93°C |
| über 1. Schuß | 93°C |
| Sumpf | 93°C |
| Wasserphase | 86 - 91 % Wasser |
| Wasser im Sumpf | 0,04 % Wasser |

Als katalytische Packung wurde Katapak®-S gefüllt mit einem sauren Ionenaustauscherharz verwendet.

Vor dem Aufstart wurde die Kolonne mit 2-Butanol gefüllt. Ein etwa 63 %iger Glucosesirup wurde eingesetzt, der etwa noch 10 % fest gebundenes Kristallwasser enthält. Das im Glucosesirup enthaltene bzw. als Kristallwasser gebundene Wasser sowie Reaktionswasser wurden mit 2-Butanol als Schleppmittel entfernt. Dieser Alkohol bildet ein heterogenes Azeotrop mit Wasser.

Nach etwa vier Stunden Versuchsdauer wurde eine Sumpfprobe entnommen und gaschromatographisch analysiert. Der 2-Butylglycosidgehalt im Sumpf betrug etwa 85 %.

### Beispiel 2

### Veretherung von Glucose mit Isopropanol

| | |
|---|---|
| Anlage | Kontinuierliche Destillationsanlage mit sechs Sektionen. Unterste Sektion und Sektion 4 und 5 mit 40 mm Durchmesser mit Sulzer CY® Packung, Sektionen 2, 3 und 4 mit 70 mm Durchmesser und katalytischer Packung. Phasentrenner am Kopf mit Rückführung der organischen Phase über Sektion 5. Glucoseeinsatz über Sektion 4 und IPA über Sektion 1. |
| Versuchsdauer | 2 Tage |
| Druck | 1015 mbar |
| Differenzdruck | 2 mbar |
| Rückfluß | 1 : 1,5 bis 1 : 5 |
| Einsätze | |
| IPA | Über 1. Schuß mit 80°C 299 g/h |
| Glucose 63 %ig | Über 4. Schuß mit 75°C 56 g/h |
| Abläufe | |
| Wasserphase | 18 - 20 g/h |
| Sumpf | 300 - 320 g/h |
| Temperaturen | |
| Kopf | 62°C |
| über 4. Schuß | 69°C |
| über 3. Schuß | 81°C |
| über 2. Schuß | 81°C |
| über 1. Schuß | 83°C |
| Sumpf | 83°C |
| Wasserphase | 87 - 88 % Wasser |
| Wasser im Sumpf | 0,01 % Wasser |

Die Kolonne vor dem Aufstart mit Isopropanol gefüllt. Als Schleppmittel für in Glucosesirup enthaltenes Wasser sowie Reaktionswasser wurde Diisopropylether verwendet. Dieses Schleppmittel entsteht in gewissem Umfang auch durch die Nebenreaktion der Veretherung von Isopropanol miteinander. Der als Edukt eingesetzte 63 %ige Glucosesirup enthielt etwa 10 % gebundenes Kristallwasser.

Eine entnommene Sumpfprobe wies einen Gehalt von 93,2 % Isopropylglykosid auf.

## Patentansprüche

1. Verfahren zum Herstellen von Glykosiden aus Sacchariden und C₁-C₈-Alkoholen, **dadurch gekennzeichnet, daß** die Veretherung durch Reaktivdestillation an einem heterogenen Katalysator erfolgt.

2. Verfahren nach Anspruch 1, **dadurch gekennzeichnet, daß** der Alkohol ein C₂-C₈-Alkohol ist.

3. Verfahren nach Anspruch 2, **dadurch gekennzeichnet, daß** der Alkohol ein C₂-C₄-Alkohol ist.

4. Verfahren nach einem der Ansprüche 1 bis 3, **dadurch gekennzeichnet, daß** der Alkohol ein primärer oder sekundärer Alkohol ist.

5. Verfahren nach Anspruch 4, **dadurch gekennzeichnet, daß** der Alkohol ausgewählt ist aus der Gruppe bestehend aus Ethanol, n-Propanol, Isopropanol, n-Butanol, 2-Butanol und sec-Butanol.

6. Verfahren nach einem der Ansprüche 1 bis 5, **dadurch gekennzeichnet, daß** der Katalysator in der Destillationskolonne fixiert ist.

7. Verfahren nach Anspruch 6, **dadurch gekennzeichnet, daß** der Katalysator ein saures Ionenaustauscherharz ist.

8. Verfahren nach Anspruch 6 bis 7, **dadurch gekennzeichnet, daß** das niedriger siedende Edukt unterhalb des Katalysators in die Destillationskollonne eingespeist wird.

9. Verfahren nach einem der Ansprüche 6 bis 8, **dadurch gekennzeichnet, daß** das höher siedende oder nicht destillierbare Edukt oberhalb des Katalysators in die Destillationskolonne eingespeist wird.

10. Verfahren nach Ansprüche 1 bis 9, **dadurch gekennzeichnet, daß** in den Edukten enthaltenes Wasser sowie Reaktionswsser mittels eines Schleppmittels abgetrennt wird.

11. Verfahren nach einem der Ansprüche 1 bis 10, **dadurch gekennzeichnet, daß** die Reaktionstemperatur bei 60 bis 200°C, vorzugsweise 60 bis 150°C, weiter vorzugsweise 70 bis 100°C liegt.

12. Verfahren nach einem der Ansprüche 1 bis 11, **dadurch gekennzeichnet, daß** ein Lösungsmittel zur Senkung der Sumpftemperatur in den Sumpf geschnitten wird.
